# EUROPEAN PATENT APPLICATION

(11) **EP 2 193 752 A1**
(43) Date of publication of application: **09.06.2010**
(21) Application number: 09177672.4
(22) Date of filing: 01.12.2009
(51) Int. Cl.: A61B 17/17

(54) **Tool**

(30) Priority: 03.12.2008 GB 0822078
(71) Applicant: Finsbury (Development) Limited, Leatherhead Surrey KT22 7BA (GB)
(72) Inventor: Spencer, John, Leatherhead, KT22 7XB (GB); Taylor, Andrew Clive, Chichester, Sussex PO19 3QQ (GB); Tuke, Michael Anthony, Guildford, Surrey GU1 3TF (GB)
(74) Representative: Smaggasgale, Gillian Helen

(57) **Abstract**

An alignment guide (1) for use in femoral head surgery comprising:
a support member (2);
a cannulated rod (16) supported by, and adjustable with respect to, the support member (2), and
two jaws, an anterior jaw (5) and a posterior jaw (6), each jaw having a proximal end connected to the support arm (2), and a distal end for clamping, in use, to the neck of the femur; at least one of said jaws being movable from a first open position to a second clamping position

## Description

The present invention relates to a tool for use in hip resurfacing operations. Morse particularly, it relates to a head alignment tool.

The efficient functioning of the hip joints is extremely important to the well being and mobility of the human body. Each hip joint is comprised by the upper portion of the femur which terminates in an offset bony neck surmounted by a ball-headed portion which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis the cartilage is removed from the acetabulum using a reamer such that it will fit the outer surface of the acetabular component of the hip prosthesis. The acetabular component can then be inserted into place. In some arrangements, the acetabular component may simply be held in place by a tight fit with the bone. However, in other arrangements, additional fixing means such as screws or bone cement may be used. The use of additional fixing means help to provide stability in the early stages after the prostheses has inserted. In some modern prosthesis, the acetabular component may be coated on its external surface with a bone growth promoting substance which will assist the bone to and thereby assist the of the acetabular in place. The bone femoral head will be removed and the femur hollowed using reamers and rasps to the prosthesis. The stem portion will then be inserted into the femur.

In some cases, a femoral component of the kind described above may be replaced with components for use in femoral head resurfacing.

Although the prosthesis being inserted when the head is being replaced or resurfaced is relatively small, the requirement for the surgeon to obtain the necessary access to the hip joint means that it is necessary to make a large incision on one side of the hip. In one technique, a straight incision is made through the skin on the posterior edge of the greater trochanter. In some techniques this incisionmay be made when the hip is flexed to 45°. By known techniques, the muscles and tendons are parted and held by various retractors such that they do not interfere with the surgeons access to the hip joint. The hip is then dislocated to provide access to the head of the femur.

It will be acknowledged that it is essential that the replacement surface for the head of the femur should be precisely located in both angular and translation positions of the axis of the femoral neck of the implant. To assist this, in some techniques, the surgeon inserts a pin in the lateral femur. The desired position of the pin will be known from pre-operative analysis of the x-rays. The surgeon will measure the desired distance down the femur from the tip of the greater trochanter and the alignment pin is inserted through the vastus lateralis fibres. The alignment pin is inserted in a transverse direction into the mid-lateral cortex and directed upwardly towards the femoral head. The pin is left protruding so that an alignment guide can be hooked over the alignment pin. Suitable alignment guides include those known as the McMinn Alignment Guide available from Midland Medical Technologies Ltd.

Prior art alignment guides of the kind described above generally comprise a hook or aperture which is placed over the alignment pin thus providing a good angular position for the axis of the implant in valgus, varus and ante-version of the neck. The guide will then be adjusted such that a cannulated rod is located such that the aperture therein is directed down the mid-lateral axis of the femoral neck. A stylus having been set to the desired femoral component size is positioned such that it can be passed around the femoral neck. When the stylus can be passed around the femoral neck, the cannulated rod is locked in position. Once the guide is stabilised in this way fine adjustments can be made until the surgeon is happy that the guide is in the required position.

A guide wire can then be inserted though the cannulated rod. This guide wire is then used in the further surgery in which the femoral head is shaped to accept the prosthesis. It will be understood that the alignment guide is an essential tool in the surgical procedure to ensure that the aperture drilled in the femoral head is both central to the femoral neck and at the correct angle of alignment to the femoral neck and that the shaping of the femoral head is accurate for the chosen head size.

It will therefore be understood that it is very important that the alignment guide is positioned correctly. Failure to do so may have the disastrous effect of allowing the machining of the cylinder of the head during the shaping procedure to "notch" into the neck of the femur. This will predispose the bone to early failure on load bearing.

Whilst the prior art alignment tools are particularly suitable for their function and have reached a high level of acceptance among surgeons, there is now a move towards a less invasive surgery in which the required incision should be as small as possible and the amount of interaction with healthy tissue is minimised. It is therefore desirable to consider carrying out femoral head replacement or resurfacing without the need to insert the alignment pin. Thus it is desirable that all of the surgical procedure takes place at the femoral head. There is therefore a requirement for an alignment guide which can function without interaction with an alignment pin.

Other guides are known which are, in use, located on the femoral neck itself. These are used in a similar manner to those described above and may involve some adjustment by the surgeon before he selects the best position.

In addition, it is desirable that the overall function and safety of the alignment guide be improved. It is further desirable that the alignment guide facilitates the accuracy and ease of use of the instruments that work from the neck.

EP 1588669 describes one example of an alignment guide which in use clamp to the neck of the femur and which enables the tool to take the correct orientation for the insertion of the guidewire from the medial neck. The guide described therein comprises:
a support member;
a cannulated rod supported by, and adjustable with respect to, the support arm; and
two jaws, a superior jaw and an inferior jaw, each jaw having a proximal end connected to the support and a distal end for clamping to the neck of the femur in use; at least one of said jaws being movable from a first open position to a second clasping position.

In one preferred arrangement, the jaws remain parallel as they are moved from the first open position to a second clamping position. In an alternative preferred arrangement biting elements are location on one or both jaws to improve the clamping of the jaw with the neck of the femus. In one arrangement, the biting element located on the inferior jaw is a toothed block.

Whilst the alignment guides of EP1588669 offer various advantages over prior art arrangements, there is a need for alternative alignment guides.

Thus according to the present invention there is provided an alignment guide for use in femoral head surgery comprising:
a support member;
a cannulated rod supported by, and adjustable with respect to, the support member; and
two jaws, an anterior jaw, and a posterior jaw, each jaw having a proximal and connected to the support and a distal end for clamping, in use, to the respective anterior and posterior sides of the neck of the femur; at least one of said jaws being movable from a first open position to a second clamping position.

The alignment guide is configured such that in use the jaws in the first open position may be passed over the head of the femur and in the second clamping position will clamp against the anterior and posterior sides of the neck of the femur.

In a preferred arrangement both jaws will be movable from the first open position to the second clamping position.

The two jaws will preferably each be movable by the operation of a screw means. Whilst each jaw may have a dedicated screw means, in a preferred arrangement the jaws will be mutually connected at their proximal ends via a screw member having two oppositely threaded ends, each threaded end being associated with a jaw such that when the screw is rotated in one direction the jaws will move towards the center of the screw to the clamped position and when it is rotated in the other direction the jaws move apart to the open position. It will be understood that in this arrangement the jaws remain parallel during the movement between the open and the clamped position.

The screw means having two oppositely threaded ends will be connected to the support member by any suitable arrangement. In one arrangement where the screw means is the screw member having oppositely threaded ends, the center portion of the screw member, which may be unthreaded, will pass through a receiving portion of the support member.

The screw means will preferably include a head to facilitate the operation of the screw means by the operator.

To improve stability of the tool, the jaws may be connected to the support member by pivot arms.

The jaws may be curved along at least a part of their length such that in use they can extend around the head of the femur and their distal ends can be clamped to the anterior and posterior sides of the of the femur. Alternatively a portion of the length will be substantially straight and in this arrangement, a portion, towards the end of the jaws, remote from the support member will be angled to allow the distal ends to clamp to the anterior and posterior sides of the neck of the femur.

The two jaws may be of the same or different configurations. Biting elements may be located on one or both jaws to improve the clamping of the jaw with the neck of the femur.

The biting element can be the same or different and may be of any suitable configuration. In one arrangement the biting element is a curved bar. The radius of curvature of the bar will generally be that which aptimises the interaction between the biting element and the femoral neck. The bar is preferably cylindrical.

The biting element, however configured, may have a pivot connection to the respective jaw to facilitate the jig being moved into position over the femoral head.

The jaws may each be of any suitable length, which may be the same or different.

The correct axis for insertion of the guide wire into the head of the femur is approximately 30 degrees from the sagittal plane and in 20 degrees anteversion. Thus the jaws and biting elements are configured such that in use the cannulated rod will be located such that a guide wire is inserted at the correct angle. In the most preferred arrangement of the present invention the tool will automatically take the correct orientation from the femoral neck.

The cannulated rod is adjustable with respect to the support member. In one arrangement, the rod is a sliding fit in the support arm. Once in the required position the cannulated rod will preferably be lockable such that once locked further movement is prevented. The locking means may be of any suitable arrangement. In one arrangement, a locking screw may be used.

The cannulated rod will in use enable the surgeon to position the guide wire. The cannulated rod may have a slot extending along at least a part of the length of the rod to assist in removing the tool from the guide wire once it is in position.

The cannulated rod will preferably have teeth located at the distal end thereof which in use can be driven into the surface of the femoral head. When driven into the head, these teeth help to clamp the alignment tool in position and to stabilise the tool.

The cannulated rod may additionally function as a measuring or gauging device and to assist this the surface of the rod may include measuring indica to assist the surgeon to know how deep they have cut.

An alignment rod support may be included on the support arm which may support one or more alignment rods which in use will provide a visual guide to assist the surgeon to check that the tool is in the correct position.

The or each alignment rod, which may be of any suitable arrangement, may be fitted into the alignment rod support by any suitable arrangement. One or more apertures may be included in the alignment rod support through which a portion of the alignment rod may be passed. The alignment rod may be a guidewire.

The tool of the present invention may additionally include stylus means of the kind known in the prior art.

The correct axis for insertion of the guide wire into the head of the femur is approximately 30 degrees from the sagittal plane and in 20 degrees of anteversion. Thus the tool of the present invention is configured such that in use the cannulated bore will be located such that the guide wire is inserted at the correct angle. The arrangement of the present invention allows the surgeon to visually check that the tool is in the correct orientation.

In femoral head resurfacing techniques, the surgeon will shape the head af the femur to fit within the cavity of the resurfacing prosthesis. This generally involves a number of shaping steps including the removal of the dome of the femoral head by means af a saw. It is important that the saw cut is made in the correct position so that an accurate fit with the prosthesis can be achieved.

The position of the cut to remove the dome of the femoral head can be calculated from the top of the dome of the femoral head. Thus a saw cutting guide may be located on the cannulated rod such that when the rod is in position, the guide will illustrate the correct position for the cut. Separate guides may be provided for each head size of resurfacing head prosthesis.

In an alternative arrangement, a saw cutting guide may be located on at least one of the jaws.

The alignment guide of the present invention may be used in combination with an elongate distal alignment guide which is described in more detail below.

The alignment guide of the present invention may be used in a method of preparing the head of a femur for femoral head resurfacing wherein the method comprises:
exposing the head of a femur;
locating the alignment guide according to the above first aspect on the neck of the femur; and
machining the head of the femur.

During the surgery, a well may be drilled into the head of the femur via the collar or rod. This well may be the definite hole diameter required of approximately 8 mm and drilled to a depth determined by the tube touching the head. A check may be made with a stylus once the tool is removed and cylinder cutters used guided over a peg placed in the well. These cutters are arranged such that the diameter cut will be correct for the head size chosen and will bottom on the top of the cut head such that the teeth of the cutter do not dangerously over-sail the head-neck junction and cause soft tissue damage or neck notching.

Thus the method preferably comprises:
exposing the head of the femur,
locating the alignment guide according to the above first aspect on the neck of the femur;
inserting a drill through the collar and drilling a well into the head of the femur;
removing the drill;
removing the alignment guide;
removing the top of the head of the femur;
inserting a guide rod into the well;
locating a sleeve cutter on guide rod and cutting the head; and
optionally chamfer cutting the head.

The correct axis for insertion of the guide wire into the head of the femur is approximately 30 degrees from the sagittal plane axis of the femur and in anteversion to allow for the natural offset in each position. Thus the tool of the present invention is configured such that in use the cannulated bore will be located such that the guide wire or drill is inserted at the correct angle. The arrangement of the present invention allows the surgeon to place, and to visually check that the tool is in the correct orientation, and position centered on the femoral head-neck junction.

It will be understood that whilst the tool of the present invention offers particular advantages for minimal invasive surgery, it can also be used in conventional surgical techniques.

The tool of the present invention, may be used with all of resurfacing head.

The present invention will now be described by way of example with reference to the accompanying figures in which:
- Figure 1: is a perspective view of the tool of the present invention;
- Figure 2: is a perspective view of the alignment tool of Figure 1 in use,
- Figure 3: is a side view of the alignment tool of Figure 1 in use;

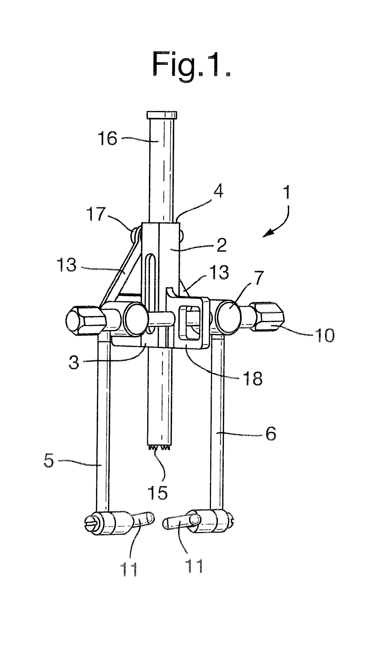

As illustrated in Figure 1, the alignment guide 1 of one embodiment of the present invention comprises a support arm 2 having a distal end 3 and a proximal end 4.

An anterior jaw 5 and a posterior jaw 6 are attached to a screw means 7 which comprises a screw member have two oppositely threaded ends and a head 10. When the head is rotated in one direction the jaws 5 and 6 move inwardly to the clamped position and when rotated in the other direction the jaws 5 and 6 move outwardly to the open position. During movement of the jaws, they remain mutually parallel.

The jaws as illustrated are straight along a majority of its length. In an alternative arrangement, they maybe curved along at least a part of their length.

The biting means on each jaw 5 and 6 is a cylindrical curved bar 11 which is located at the end of the jaw and perpendicular thereto.

The jaws are additionally connected to the support means 2 by pivot arms 13.

The cannulated rod 16 is a sliding fit in a sleeve in the support means. A locking screw 17 will enable the user to lock the cannulated rod at the required position. A bore will extend through the rod. Teeth 1 are located on the face of the bore.

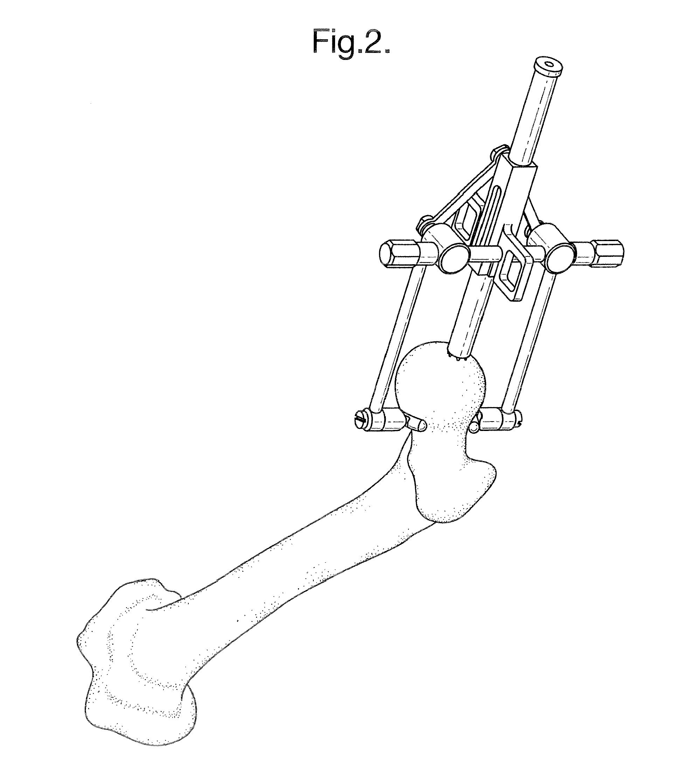

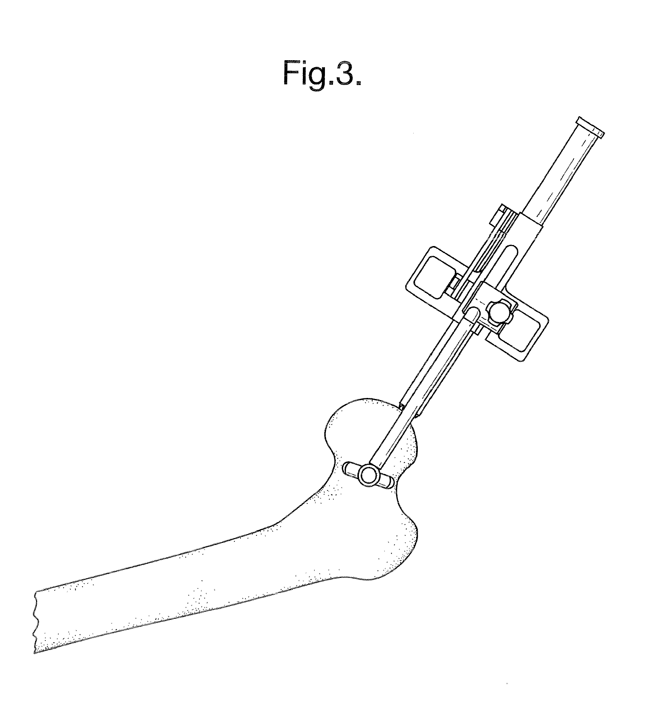

The tool of the present invention in the clamped position on the of a femur is illustrated schematically in Figures 2 and 3.

The alignment guide of the present invention may be used in combination with an elongate distal alignment guide. The elongate distal alignment guide is used to suggest an optimum femoral component angle for the resurfacing head implant. The alignment guide of the present invention suggests an angle for insertion of the guide wire and ultimately the final implanted femoral resurfacing head prosthesis, relative to the leg alignment axis. The leg alignment axis is a theoretical line between the centre of the femoral head, middle of the knee and middle of the ankle when the person is standing. The axis can be measured easily between the femoral head and knee on a patient in surgery.

A flag holder 18 having apertures (not shown) through which a flag such as a guide wire may be passed.

The elongate distal alignment guide may be attached to the alignment guide of the present invention or it may be a separate component which may be connectable to the alignment guide of the present invention or separate therefrom. Where it is separate, it will touch on the elongate guide of the present invention to measure the current femoral component angle.

## Claims

1. An alignment guide (1) for use in femoral head surgery comprising:
a support member (2);
a cannulated rod (16) supported by, and adjustable with respect to, the support member (2), and
two jaws, an anterior jaw (5) and a posterior jaw (6), each jaw having a proximal end connected to the support arm (2), and a distal end for clamping, in use, to the neck of the femur; at least one of said jaws being movable from a first open position to a second clamping position.

2. An alignment guide according to Claim 1 wherein both jaws (5, 6) are movable from the first open position to the second clamping position.

3. An alignment guide according to Claim 1 or 2 wherein the jaws (5, 6) remain parallel as they move from the first open position to the second clamping position.

4. An alignment guide according to any one of Claims 1 to 3 wherein the two jaws will each be movable by a screw means.

5. An alignment guide according to Claim 4 wherein the two jaws are mutually connected at their distal ends via a screw member having two oppositely threaded ends.

6. An alignment guide according to Claim 5 wherein the center portion of the screw member passes through a receiving portion of the support member and wherein the center portion of the screw is optionally unthreaded.

7. An alignment guide according to any one of Claims 1 to 6 wherein the jaws (5, 6) are connected to the support member (2) member by pivot arms (13).

8. An alignment guide according to any one of Claims 1 to 7 wherein the jaws (5, 6) are curved or straight along at least a part of their length.

9. An alignment guide according to any one of Claims 1 to 8 wherein biting elements (11, 12) are located on one or both jaws (5, 6) to improve the clamping of the jaw with the neck of the femur.

10. An alignment guide according to Claim 9 wherein the biting element on each jaw is an optionally pivotable bar (11) located at or near the proximal end of the jaw.

11. An alignment guide according to any one of Claims 1 to 10 wherein the cannulated rod (16) is adjustable with respect to the support arm (2).

12. An alignment guide according to Claim 11 wherein the rod (16) is a sliding fit in the support arm (16).

13. An alignment guide according to Claim 11 or 12 wherein the tool additionally includes (17) means for locking the cannulated rod with respect to the support member.

14. An alignment guide according to any one of Claims 1 to 13 wherein the alignment guide additionally includes one or both of a saw cutting guide and a flag holder (18).

15. An alignment guide according to any one of Claims 1 to 14 wherein in use the location of the jaws on the femoral neck will cause the bore of the cannulated rod to be aligned with the central axis of the femoral head and neck.

16. An alignment guide according to Claim 15 wherein the axis is approximately 30 degrees from the sagittal and in 20 degrees of anteversion,

17. A kit comprising the alignment guide of any one of Claims 1 to 18 and an elongate distal alignment guide (20) which by referencing the back of the knee will reference the leg alignment axis.
